# EUROPEAN PATENT APPLICATION

(11) **EP 3 839 960 A1**
(43) Date of publication of application: **23.06.2021**
(21) Application number: 19217329.2
(22) Date of filing: 18.12.2019
(51) Int. Cl.: G16H 10/60, G16H 20/00, G16H 50/20

(54) **GENERATING CONTEXTUALLY-USEFUL GUIDANCE FOR TREATING A PATIENT**

(71) Applicant: Koninklijke Philips N.V., 5656 AG Eindhoven (NL)
(72) Inventor: EERDEN, Jacco Christof, 5656 AE Eindhoven (NL); DANISMAN TASAR, Mine, 5656 AE Eindhoven (NL); HAVERSTOCK, Christopher Edward, 5656 AE Eindhoven (NL)
(74) Representative: Philips Intellectual Property & Standards

(57) **Abstract**

A process for producing patient-specific guidance. Generic guidance is processed, based on patient-specific data, to thereby generate patient-specific guidance that can be used to improve an understanding of an audience member (of the patient-specific guidance) to medical subject-matter associated with the patient.

## Description

### FIELD OF THE INVENTION

The present invention relates to the generation of guidance for treating a patient, such as the generation of guidelines, protocols, treatment strategies and/or operating procedures.

### BACKGROUND OF THE INVENTION

Due to the inherently complex nature of the medical field, caregivers can often find it difficult to decide upon a treatment strategy for a patient.

There has been a growing trend in healthcare, to support clinical decision making, to make use of guidance for treating a patient, such as guidelines, protocols, treatment strategies and/or operating procedures. This guidance is typically produced by nationally or internationally recognized bodies or medical content providers and is made accessible by a client. The guidance may be kept up to date by these bodies regularly pushing or otherwise providing updated guidance.

However, the present invention recognizes that this guidance is typically generic and a clinician is therefore required to use their own medical knowledge and professional judgment to adapt the guidance for the patient. This can introduce error into the treatment of the patient, as treatment can deviate from a recommended process.

This is a particular problem for patients presenting unusual or atypical signs/symptoms and/or with inexperienced clinical staff.

There is therefore a desire to overcome at least some of these problems that occur when treating patients based on generic guidance for treating a patient.

### SUMMARY OF THE INVENTION

The invention is defined by the claims.

According to examples in accordance with an aspect of the invention, there is provided a method of generating patient-specific guidance for treating a patient.

The method comprises: obtaining patient-specific data associated with a patient; obtaining generic guidance providing generic guidelines, protocols, treatment strategies and/or operating procedures for treating the patient; and processing the generic guidance, using one or more computer-based rules or algorithmic processes, based on the patient-specific data to generate patient-specific guidance to thereby provide patient-specific guidelines, protocols, treatment strategies and/or operating procedures for treating the patient.

Thus, generic guidance is adapted for a specific user based on the patient-specific data associated with the user. This results in the production of more helpful, relevant and appropriate guidance that can aid in the treatment of the patient. The clinician is thereby able to access patient-specific guidance that credibly assists the clinician in making a clinical decision, e.g. diagnosing or treating the patient. This thereby improves the long-term health of the patient, as significant improvements are made to the ability of the clinician to appropriately treat the patient.

In particular, guidance is contextualized or customized to the condition or characteristics of the patient. This reduces an amount of time and labor spent by a clinician in adapting the generic guidance to the patient's specific needs and requirements.

Processing the generic guidance may comprise modifying or adapting content of the generic guidance to generate the patient-specific guidance. This effectively provides dynamic guidance that adapts to the patient.

The generic guidance may comprise generic guidance for: performing a particular treatment/clinical procedure (e.g. intubation guidance or cricothyroidotomy guidance), providing medication (e.g. guidance for providing respiratory drugs or cardiovascular drugs), addressing certain patient characteristics (e.g. guidance for handling electrolyte abnormalities or nutrition) and/or clinical management guidelines (e.g. guidance for withdrawing treatment in the ICU or preparing a patient for mobilization). Generally speaking, the generic guidance is any suitable guidance, instruction set, protocol, treatment strategy or operating procedure for handling a patient using good clinical practices for maintaining, improving or ensuring the patient's ongoing health.

The method may comprising a step of obtaining clinician information associated with a clinician attending the patient, wherein the step of processing the generic guidance comprises: processing the generic guidance, using one or more computer-based rules or algorithmic process, based on the patient-specific data and the clinician information to generate patient and clinician specific guidance to thereby provide patient and clinician specific guidelines, protocols, treatment strategies and/or operating procedures for treating the patient.

Thus, the generic guidance may be further contextualized to information about the clinician, to maximize the relevance and appropriateness of the patient-specific guidance to the clinician. This improves the generic guidance.

Preferably, the patient-specific data comprises real-time patient monitoring data. This ensures that the patient-specific guidance is up-to-date, and can take account of a current or ongoing status of the patient.

The patient-specific data may comprise an electronic medical record of the patient. This embodiment takes advantage of stored data of the patient in order to improve the contextualization of the patient-specific guidance.

The method may comprise a step of displaying a visual representation of the patient-specific guidance. This enables the patient-specific guidance to be presented to the clinician, to enable them to make a clinical decision (e.g. select a treatment strategy) based on the patient-specific guidance.

In some embodiments, the method further comprises, after displaying the visual representation, responding to a user input to update the patient-specific guidelines and/or the patient-specific data. In this way, a clinician is able to modify the patient-specific guidelines (e.g. to indicate an intended change in the treatment strategy) or the patient-specific data (e.g. to indicate an adherence to guidance). This ensures that the system is able to monitor the adherence of staff to the guidance, which can help with later review for optimizing the care delivery procedure.

The step of obtaining generic guidance may comprise processing patient-specific data, using one or more computer-based rules or algorithmic processes, to identify generic guidelines for the patient. Thus, appropriate (albeit generic) guidelines may be automatically selected by processing the patient data to identify appropriate generic guidelines for the patient. This may take place by, for example, identifying generic guidelines for treating a certain condition of the patient (which may be indicated in the patient data).

In some embodiments, the step of obtaining the patient-specific data comprises obtaining the patient-specific data from patient-specific data module comprising a first, different database, a user interface, and/or a patient monitoring device; and the step of obtaining the generic guidance comprises obtaining the generic guidance from a second database.

The step of obtaining the generic guidance optionally comprises responding to a user input indicating desired generic guidance to obtain the generic guidance from the second database. Thus, a clinician may be able to control the generic guidance that is processed to generate patient-specific guidance.

The step of processing the generic guidance comprises selecting a sub-set of the generic guidelines, protocols, treatment strategies and/or operating procedures provided by the generic guidance based on the patient-specific data, to thereby generate patient-specific guidelines, protocols, treatment strategies and/or operating procedures.

Thus, generic guidance may undergo a filtering process in order to identify appropriate parts or portions of the generic guidance that are suitable for the patient. This enables content irrelevant to the patient to be excised from the generic guidance.

In some embodiments, the generic guidance comprises a procedural checklist for a treatment of the patient, wherein the step of processing the generic guidance comprises populating the checklist based on the patient-specific data.

In some embodiments, the generic guidance comprises a decision tree for aiding a clinician in making a diagnosis, wherein the step of processing the generic guidance comprises incorporating patient-specific data into the decision tree.

In some embodiments, the generic guidance comprises a proposed treatment strategy for the patient, wherein the step of processing the generic guidance comprises adapting the treatment strategy based on the patient-specific data.

Any combination of these and other options for the generic guidance are envisaged by the present invention.
According to examples in accordance with an aspect of the invention, there is provided a computer program product comprising computer program code which, when executed by one or more processing systems, cause the one or more processing systems to perform all of the steps of any herein described method.

According to examples in accordance with an aspect of the invention, there is provided a processing system for providing patient-specific guidance for treating a patient.

The processing system is adapted to: obtain patient-specific data associated with a patient; obtain generic guidance providing generic guidelines, protocols, treatment strategies and/or operating procedures for treating the patient; and process the generic guidance, using one or more computer-based rules or algorithmic processes, based on the patient-specific data to generate patient-specific guidance to thereby provide patient-specific guidelines, protocols or operating procedures for treating the patient.

An additional advantage of the present invention is the patient specific guidelines are provided at once without a laborious database search. In common practice the clinician (user) would not only need to review recent data stored in the electronic medical records (which are not even real-time data) but in parallel search internal database for a vast plurality of existing protocols, which need to be aligned further aligned with generic medical practice.

There is also proposed a patient-specific guidance system comprising the processing system previously described; a patient-specific data module adapted to provide patient-specific data, wherein the patient-specific data module comprises a first database, a user interface and/or a patient monitoring device; a second, different database adapted to store generic guidance providing generic guidelines, protocols, treatment strategies and/or operating procedures for treating the patient; wherein the processing system is adapted to obtain the patient-specific data from the patient-specific data module and the generic guidance from the second, different database.

There is also proposed a patient-specific guidance display system comprising: a processing system previously described or a patient-specific guidance system previously described; and a user interface adapted to provide a visual representation of the patient-specific guidance information generated by the processing system.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 conceptually illustrates an embodiment of the invention;
Figure 2 illustrates a patient-specific guidance display system for displaying patient-specific guidance;
Figure 3 illustrates another example of a patient-specific guidance display system for displaying patient-specific guidance; and
Figure 4 is a flowchart illustrating a method according to an embodiment of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a process for generating patient-specific guidance for treating a patient. In particular, the methods relates to a concept for modifying or adapting generic guidance for treating a patient based on patient-specific data. This enables the generation of patient-specific guidelines, protocols, treatment strategies and/or operating procedures for treating the patient.

Embodiments may be employed in a clinical setting, to aid in the treatment of a patient within the clinical setting. Alternatively, embodiments may be employed remotely from the clinical setting to aid a remote clinician is selecting or choosing a treatment strategy for the patient.

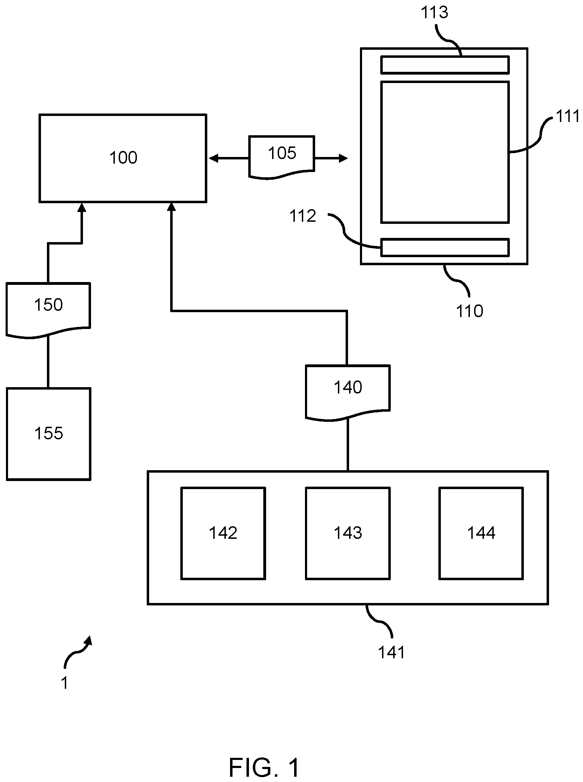

Figure 1 illustrates a processing system 100 according to an embodiment of the invention. The processing system 100 is explained in the context of a patient-specific guidance display system 1, which is itself an embodiment of the invention.

The processing system 100 generates patient-specific guidance 105 from patient-specific data 140 and generic guidance 150. In other words, the generic guidance is contextualized using the patient-specific data to provide more relevant and tailored guidance. This helps improve the applicability of the guidance to aid a clinician in making a clinical decision on a treatment strategy for the patient, i.e. aid the clinician in treating the patient.

The patient-specific guidance 105 is then passed to a destination device. In the illustrated examples, the destination device is a user interface 110 for displaying (a visual representation of) the patient-specific guidance. In other examples, the destination device is a server or storage facility (not shown), e.g. for later accessing by a user interface.

The patient-specific guidance may therefore be displayed at or otherwise output by one or more user interfaces 110 of the guidance display system 1. In particular embodiments, the user interface(s) 110 may provide a visual representation of the patient-specific guidance, e.g. at a screen 111. The user interface 110 may also provide an input mechanism 112, e.g. a keyboard or a touch-sensitive screen, for enabling a user to provide a user input (embodiments of which will be later explained).

The processing system 100 is adapted to receive patient-specific data 140 from a set patient-specific data module 141, which comprises one or more data sources 142-144. These data sources can be a plurality of medical sensors coupled to the patient. In this case the patient specific data module can be a patient monitoring device associated with the present patient. Therefore, the patient-specific data 140 would be received real-time and enable the user to get an instantaneous guideline representative of the patient's state. In another embodiment, the patient monitoring device associated with the patient can be one of the data sources 142-144, while other sources would be one of or a combination of, for example, a data base of lab tests, historical information from an electrical medical records. Any of these elements may form part of the overall guidance display system 1.

A first data source 142 may be a (first) database. The (first) database 142 may, for example, store information about a specific patient, such as the electronic medical record of the patient.

A second data source 143 may be a user interface. The user interface 143 may allow a user to directly input information about the patient to the processing system 100. This may, for example, be in the form of answers to a questionnaire for defining the patient-specific data. In embodiments, the second data source 143 comprises or is formed as an aspect of the user interface 110.

A third data source 144 may be a patient monitor. Use of a patient monitor enables real-time monitoring data of the patient to influence the patient-specific guidance, thereby ensuring that the patient-specific guidance is up to date.

Thus, the patient-specific data may comprise any combination of: medical history information (e.g. from the first/second data source); patient demographic information (e.g. from the first/second data source) and/or real-time medical monitoring information (e.g. from the third data source). Other examples of patient-specific data would be apparent to the skilled person.

Any combination of one or more of these data sources (and possible options for the patient-specific data) may be used, according to various embodiments.

By way of further, non-exhaustive example, the patient-specific data may comprise: real-time vital signs and other system organ/physiological measurement (i.e. hemodynamics, respiratory, infections, delirium) of the patient; averaged values of the same (e.g. daily averaged values); a patient record (e.g. electronic medical record) providing demographic information (age, weight, gender etc.), medical history, diagnosis information and so on; and/or data about lab results, medication, IV fluids, mechanical ventilation and so on.

The processing system 100 is adapted to receive generic guidance 150 for treating a patient, for example, from a (second, different) database 155.

The generic guidance provides generic guidelines, protocols, treatment strategies and/or operating procedures for treating the patient. The generic guidance may be guidance for treating a single condition/diagnosis (e.g. a specific condition/diagnosis associated with the patient), performing a particular treatment step (e.g. guidance for performing intubation), providing medication (e.g. guidance for providing respiratory drugs) or for treating a range of different conditions/diagnoses.

The obtained generic guidance may be based on a user input (e.g. provided at the user interface 110). Thus, the clinician or other user may select the generic guidance that is obtained (e.g. from the database 155). For example, the user may select generic guidance on how to perform intubation, or guidelines on providing certain drugs.

In some examples, the obtained generic guidance may be based upon the patient-specific data 140. For example, the patient-specific data 140 may provide an indication of one or more diagnoses associated with the patient, and the obtained generic guidance may be guidance for treating the indicated diagnosis or diagnoses. In another example, the patient-specific data 140 may provide an indication that the patient is getting ready to leave the clinical setting, and the generic guidance may comprise guidance for preparing the clinician to leave the clinical setting (e.g. a checklist of criteria that must be met before the patient is permitted to leave).

Of course, a combination of these approaches may be used. For example, patient-specific data may be used to identify a set of generic guidances for the patient (e.g. based on their diagnoses or patient history), and a user input may be used to select one of these guidelines for use as the generic guidance.

Other methods of defining or selecting the generic guidance 150 will be apparent to the skilled person.

The processing system 100 processes the generic guidance, based on the patient-specific data, in order to produce the patient-specific guidance. The processing system 100 performs this step using one or more computer based rules or algorithmic processes.

In particular, the content of generic guidance is adapted based on the patient-specific data, to produce patient-specific guidance having content that is adapted to the specific patient.

An example of a suitable computer-based rule or algorithmic process is a neural network or other machine learning algorithm.

A machine-learning algorithm is any self-training algorithm that processes input data in order to produce or predict output data. Here, the input data comprises generic guidance and patient specific data, and the output data comprises patient-specific guidance.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian model are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to examples the patient data and corresponding generic guidance. The training output data entries correspond to corresponding examples of patient-specific guidance.

Other rules or algorithmic process may be employed by the skilled person, and may depend upon the format of the generic guidance.

For example, generic guidance may comprise a template for a treatment recommended for the patient, and the patient-specific data may be used to complete or fill out missing portions of the template. The template may, for example, comprise equations identifying a relationship between patient characteristics and treatment guidelines (e.g. calculating a recommended dosage based on patient characteristics), which may be executed based on the patient-specific data.

Preferably, the processing of the generic guidance comprises filtering the generic guidance using the one or more computer-based rules or algorithmic processes.

For example, generic guidance for treating a patient may list different options depending upon characteristics of the patient. In one example, different dosages of medication could be recommended for different patient ages/weights/genders. The patient-specific data may be used to remove irrelevant or unnecessary information (e.g. information that does not correspond to characteristics of the patient, such as information for a different gender). This makes the patient-specific guidance more relevant and appropriate for the patient.

By way of example only, generic guidance may contain two versions of a certain aspect of guidance, a first version pertinent to patients at or over the age of 65 and a second version pertinent to patient under the age of 65. The first version of this certain aspect may be filtered out if the patient is under the age of 65 (as indicated in the patient-specific data), to provide the more relevant second version as an aspect of the patient-specific guidance.

By way of yet another example, generic guidance may contain different guidance portions for multiple different possible conditions of the patient. The one or more rules/algorithms may process the patient-specific data to identify any conditions of the patient and select only those guidance portions associated with any identified condition of the patient to include in the patient-specific guidance.

Thus, there may be a larger body of generic guidance that is filtered (i.e. elements removed) by the one or more computer-based rules or algorithmic processes to generate the patient-specific guidance. This effectively enables the guidance to be prepared in advance (in the generic guidance), with only the patient-appropriate portions of the generic guidance being provided in the patient-specific guidance.

This provides a simple and low processing demand method of generating patient-specific guidance from a large body of generic guidance.

In preferred examples, the generic guidance contains a list of items or elements that are associated with a particular treatment step, treatment strategy or clinical guideline. The patient-specific data may be used to mark off or otherwise identify which of these items or elements have been performed (or are otherwise associated) with the patient.

By way of example, guidance for performing an intubation step may include guidance on medication to be supplied for intubation (e.g. to prevent infection, anaesthetize/sedate the patient, to relax muscles, for pain-relief and so on). The patient-specific data may identify medications already provided to the patient (e.g. in the patient record) and automatically identify or flag the medications that have been provided. This results in the generic guidance being adapted based on the patient-specific data to provide patient-specific guidance.

The processing system 100 may be adapted to receive additional information, to assist in the generation of the patient-specific guidance.

The additional information may be used as an additional input for the computer-based rules and/or algorithms (e.g. new features for a neural network).

In other examples, the additional information may be used to control a mode setting for the processing system 100, e.g. to define which rules and/or algorithms, aspects of the input data or aspects of the possible output data (for the rules/algorithms) are used when generating the patient-specific guidance. Examples of mode settings will be described later.

The additional information may, for example, comprise clinician information, being information about and/or identifying the clinician.

Information about the clinician may, for example, be obtained from a user interface 110 which could allow a user to input information about themselves or could use log-in information to identify the clinician. In other embodiments, information about the clinician could be obtained from a camera (not shown), e.g. by performing a facial recognition process on images/videos obtained by the camera.

Embodiments employing information about the clinician enables the patient-specific guidance to be contextualized for that clinician, thereby improving the relevance of the treatment information

Clinician information may comprise, for example, information about: an identity of the clinician, a job role of the clinician, a knowledge level of the clinician; an experience of the clinician; an error-rate of the clinician and so on.

Consider an example in which responsibility for a patient's treatment is divided between two different clinicians, who have different areas of responsibility (e.g. a cardiologist may be responsible for the patient's heart health, and an urologist for their urinary tract). The processing system 100 may be adapted to use the clinician information (e.g. identifying a job-role of the clinician) to generate patient-specific guidance that pertains to the area of responsibility of the clinician.

As another example, consider a scenario in which different clinicians are qualified to perform certain treatment tasks on the patient (e.g. qualified to perform intubation or qualified to operate a continuous positive airway pressure (CPAP) device). The processing system 100 may be adapted to use the clinician information (e.g. identifying qualifications of the clinician) to generate patient-specific guidance that provides guidance for treatment tasks for which the clinician is qualified to perform for treating the patient.

Other additional information may be used depending upon the circumstances, such as information about the clinical setting or available equipment (for performing a treatment).

By way of example, the additional information may comprise equipment information that identifies equipment that is available to be used when treating the patient. If the generic guidance for treating the patient allows different pieces of equipment to be used to perform a certain treatment task, then the equipment information may be used to remove information about pieces of equipment that are not available to be used.

By way of another example, a clinical setting may have the capability to perform certain treatment (e.g. a surgery for performing a surgical treatment). In this scenario, if the generic guidance for treating the patient offers the option of a surgery, then clinical setting information may be used to determine whether this is an option within the context of the clinical setting, and either maintain/leave or remove/excise the relevant portion of the generic guidance.

The above examples and scenarios are non-exhaustive, and are used to demonstrate the flexibility of the proposed system for adapting generic guidance for treating the patient based upon the context of the patient (e.g. their clinicians or their environment).

Although some examples for processing the generic guidance have been described, the precise operation performed on the generic guidance, e.g. to generate patient-specific guidance, may depend upon the nature and/or format of the generic guidance.

For example, the generic guidance may comprise instructions or suggestions treating a patient, e.g. options for possible interventions, whether or not to perform fluid management, options on medications for the patient and so on. The patient-specific data may be used to refine these instructions or suggestions based on one or more computer-based rules or algorithmic processes. For example, the patient-specific data may be used to provide suggestions for a type of intervention (e.g. which option of possible interventions should be used), information on fluid management (e.g. how much fluid and how regularly fluid should be provided), and/or suggested medications and dosages. These options may be adjusted based on the patient-specific data, such as the patient's age, weight, length, (hemodynamic) status, past medication.

In another example, the generic guidance may comprise a "decision tree" that provides a visual representation of the patient's condition and a decision-making process for a treatment option. The "decision tree" may be updated with the patient data, e.g. in "real-time", to aid the clinician in making a diagnosis and/or treatment selection.

In yet another example, the generic guidance may comprise a checklist for performing a certain treatment strategy, the checklist identifying tasks performed to carry out the treatment strategy. The processing system 100 may automatically mark tasks as "complete" based on the patient-specific data, e.g. based on a patient history that would record treatment tasks that have already been performed.

In another example, the generic guidance may provide clinicians with workflow guidance (e.g. identifying events or tasks that need to be performed in response to patient data) and remind the clinician to perform certain tasks based on patient data.

These examples are non-exhaustive and only serve to further clarify and emphasize the flexibility and applicability of the underlying concept, namely that of modifying guidance (information) based on patient-specific data.

The patient-specific guidance may be updated or adapted. This may be performed by either re-generating the patient specific guidance using the generic guidance (i.e. "updating" the patient-specific guidance) or further processing the patient-specific guidance using at least patient-specific data (i.e. "adapting" the patient-specific guidance).

Updating the patient-specific guidance may comprise, for example, performing any previously described process to generate new patient-specific guidance. This enables the patient-specific guidance to be re-generated based on, for example, changes in the patient-specific data.

Adapting the patient-specific guidance may comprise, for example, processing both the patient-specific guidance and patient-specific data, using one or more computer-based rules or algorithms, to adapt the patient-specific guidance. This enables the patient-specific guidance to be adapted based on, for example, changes in the patient-specific data, and may reduce a processing cost compared to a step of updating the patient-specific guidance.

An in-depth description of how to process the patient-specific guidance and patient-specific data to adapt the patient-specific guidance will not be described, for the sake of conciseness, as the skilled person would readily understand how such processing methods could be implemented (e.g. by consulting the previous description on how the patient-specific guidance may be initially generated).

Updating or adapting may be performed automatically or periodically, in response to detection of a change of state of the user interface 110 or processing system 100, detection of a change in the patient-specific data and/or in response to a user input.

In one example, patient-specific guidance is updated and/or adapted at periodic intervals, e.g. once a day, once every 12 hours, once every 4 hours and so on.

In another example, patient-specific guidance may be updated and/or adapted to coincide with shift changes of a caregiver (e.g. by consulting a calendar or monitoring the movement of caregivers throughout a clinical setting, e.g. using a camera or badge tracking system).

In another example, patient-specific guidance is updated or adapted in response to detecting a state of the user interface, e.g. in response to a new user interacting with (e.g. logging in) to a user interface.

In some examples, the patient-specific guidance may be updated (e.g. the process for generating such content repeated) in response to a user input. Thus, a user interface 110, 142 may be adapted to generate a user input (responsive to an action of the user) and pass this user input to the processing system 100.

In further embodiments, the process of updating or adapting the patient-specific guidance is further based on this user input. Thus, a user input may act as an additional input or feature for the computer-based rules or algorithms in generating the patient-specific guidance. This enables the user to direct the updating/adapting of the patient-specific guidance.

In embodiments in which a user input triggers the updating or adapting of the patient specific guidance, the processing system may be adapted to process the patient-specific guidance and the user input, using one or more computer-based rules or algorithms, based on the user input to generate adapted patient-specific guidance. Thus, there may be no need to use the patient-specific data in adapting the patient-specific guidance. This allows the user to direct how the patient-specific guidance is adapted or filtered.

In some examples, patient-specific guidance is updated or adapted in response to an update or change in the patient-specific data. It will be apparent that the patient-specific data can be automatically or manually updated over time. For example, patient-specific data may comprise real time patient monitoring data, such as heartrate or other vital sign information.

In some examples, the patient-specific guidance is updated or adapted in response to a significant update or change in the patient-specific data. A significant change or update may, for example, be an update or change that indicates a change in a value of the patient-specific of more than a predetermined percentage (e.g. more than 5% or 10% change in the patient-specific data). In other examples, a significant change or update might be a change or update of the patient-specific data that results in a one or more values of the patient-specific data breaching predetermined (clinically acceptable) thresholds.

It will therefore be clear that a "significant update or change" may indicate a medically significant change or update in the patient-specific data, e.g. a change that might require clinical attention or a change that would affect a clinical decision for the patient. The rules for whether such a change is significant may be predefined, e.g. in clinical compliance guidelines or the like.

Of course, a combination of these features may be performed. For example, the patient-specific guidance may be updated or adapted periodically, but only if there has been a significant update or change in the patient-specific data. In another example, the patient-specific guidance may be updated/adapted in response to a user input, but only if a certain time period has elapsed since the last update/adaptation. Such embodiments may save or reduce unnecessary processing power.

It will be apparent that a user or operator of the processing system may be able to modify and control the updating/adapting policy, e.g. whether, how and when the patient-specific guidance is updated or adapted.

It has previously been described how a user interface 110 may be used to display or otherwise output the patient-specific guidance.

The user interface 110 may, for example, comprise a screen 111 for displaying the patient-specific guidance, as would be well known to the skilled person. The user interface 110 may comprise a processor 110 adapted to process the patient-specific guidance (e.g. textual data or graphical information) to generate display data for controlling the screen 111 to provide a visual representation of the patient-specific guidance.

Suitable methods of controlling and displaying guidance via a user interface will be apparent to the skilled person.

In some examples, the user interface comprises a speaker 113 for providing an audio output of the patient-specific guidance. This audio output may be performed, for example, by performing a text-to-speech operation on textual information of the patient-specific guidance, or by the patient-specific guidance itself comprising an audio file.

As previously explained, the user interface may comprise an input mechanism 112 for enabling a user to provide a user input. The input mechanism may comprise any known input mechanism, e.g. a mouse or keyboard. In some examples, the input mechanism is integrated with the screen 111, e.g. a touch-sensitive screen.

The user interface 110 may be integrated or an aspect of, for example, a bedside monitor, a workstation, a mobile device (e.g. mobile/cellular smartphone, smartwatch, tablet or smart glasses), a projection device, a laptop, a computer and so on.

In some embodiments, the user interface 110 may be adapted to allow an operator of the user interface, such as a clinician, to provide additional information about the patient (e.g. in response to the patient-specific guidance). This may be provided via the input mechanism 112.

This additional information about the patient may be used to supplement the patient-specific data originally used to generate the patient-specific guidance. The additional information may (also or alternatively) be made available to clinical staff to assist in the clinical staff making a clinical decision.

In some embodiments, the additional information generated by the user interface is passed to the patient-specific data module 151, and in particular embodiments, to the second database of the patient-specific data module 152. Information in this second database 152 may be accessible by clinical staff in order to aid them in making a clinical decision.

In particular, the user interface may allow a clinician to report or record adherence to guidance for treating the patient, e.g. by recording actions taken. This information can, as previously be explained, be written back into the patient-specific data module (e.g. the patient's electronic medical record).

In some examples, recording adherence to guidance for treating the patient is performed automatically, e.g. by sensors or patient monitoring devices. For example, guidance may indicate that a step of attaching electrodes to a patient should be performed, and a patient monitoring device may be adapted to detect when a step of attaching electrodes has been performed and provide information (e.g. written to an electronic medical record) that this step has been performed. This enables automatic updating of the patient-specific information responsive to the patient-specific guidance.

In other words, the processing system 100 may be further configured to determine an adherence to the patient-specific guidance, generate adherence information responsive to the determined adherence, and store the adherence information in a patient-specific data module (e.g. in the patient's electronic medical record).

This additional information may be used, in some example, to update or adapt the patient-specific guidance, although this is not essential.

The herein proposed embodiments thereby enable the provision of improved and more relevant information for assisting in making a clinical decision (e.g. diagnosing or treating) a patient.

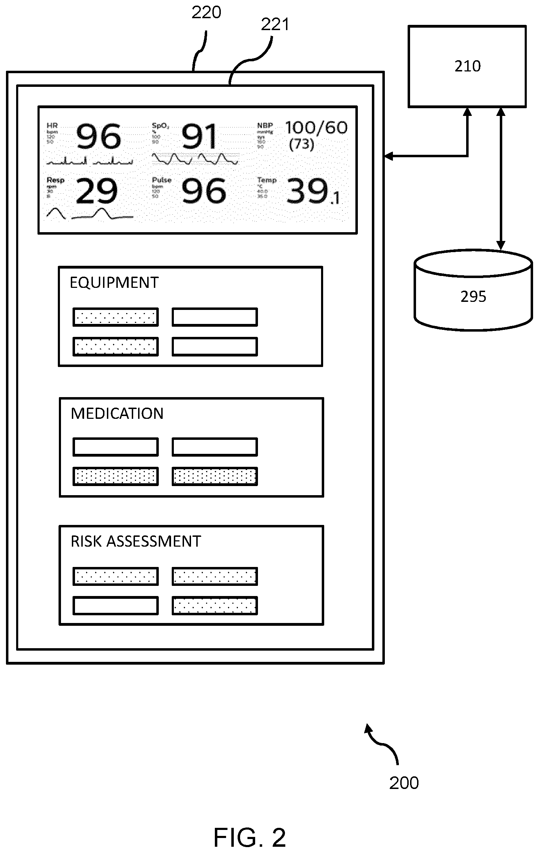

A working example of an embodiment of the invention will be hereafter described with reference to Figure 2, which illustrates a patient-specific guidance display system 200 for displaying patient-specific guidance.

The illustrated patient-specific guidance display system 200 is integrated into a bedside patient monitor, but may be formed as an aspect of any suitable electronic device, e.g. a smartphone, a tablet, a smartwatch, smart glasses and so on.

The patient-specific guidance display system 200 comprises a processing system 210 and a user interface 220. The processing system 210 is adapted to generate the patient-specific guidance and the user interface is adapted to provide a visual representation of the patient-specific guidance, e.g. at a screen 221.

The user interface 220 is also adapted to enable a user to communicate with the processing system 210. In particular, the user interface may display an interactive visual representation with which the user can interact, e.g. via an input mechanism such as a touch-sensitive screen and/or mouse/keyboard.

The user interface 220 is here configured to enable a user to select generic guidance, such as a standard operating procedure (SOP) or a guideline for performing a certain treatment step. In particular, the selection is provided to the processing system 210, which then obtains the selected generic guidance (e.g. from a database 290).

The processing system 210 also obtains patient-specific data (e.g. from another database 295) which is used to modify or adapt the generic guidance obtained from the database 290, thereby generating the patient-specific guidance.

In the hereafter described example, the generic guidance is a standard operating procedure for performing an intubation step, which takes the form of a checklist. The generic guidance may have been obtained responsive to a user input (e.g. a user selecting an "Intubation Guidance" option).

The illustrated checklist is here formed in three sections: "Equipment", which details the types of equipment required for performing intubation; "Medication", which details types and dosages of medication recommended for intubation; and "Risk Assessment", which identifies patient characteristics that contribute to a patient's risk (e.g. Mallampati score).

One or more of these sections may be omitted, according to various embodiments, and/or additional sections may be included.

The patient-specific data is used to modify or adapt the generic guidance to reflect a condition, history and/or characteristics of the patient. This may comprise, for example, marking or identifying steps of the checklist (provided by the guidance) based on a patient history and/or modifying proposed steps of the checklist based on patient characteristics or a patient condition.

In the illustrated example, the patient-specific data is used to mark or identify which elements of the checklist forming the different sections of the generic guidance have already been completed, are already available or are otherwise already associated with the subject. This is conceptually illustrated using hatching.

Various examples of adapting the generic guidance on intubation based on the patient-specific data will now be described.

For example, with reference to the "Equipment" section of the checklist, elements of the checklist for the generic guidance may be modified based on patient characteristics. For example, a size/diameter of an intubation tube recommended for an intubation step may be modified based on patient characteristics (e.g. patient height/weight/age).

As another example, with continued reference to the "Equipment" section of the checklist, elements of the checklist may be identified as present or complete based on patient history information. For example, if guidance recommends that a patient should be provided with intravenous drugs, and the patient history indicates that the patient is already fitted with an intravenous cannula, then this piece of equipment may be marked or checked off automatically.

As another example, generic guidance may indicate under what circumstances (e.g. which treatments and/or patient characteristics) a particular piece of equipment is used, and the patient-specific data may be used to select the pieces of equipment required by the patient. This may comprise, for example, identifying whether mechanical ventilation is required or not based on the patient-specific data, and including or excluding a mechanical ventilator from the equipment checklist. Thus, elements may be omitted from the checklist if they are not relevant for the patient.

As yet another example, the "Medication" checklist may indicate recommended medications for performing the intubation step, e.g. medications for preventing infection, anaesthetizing/sedating the patient, relaxing muscles, for pain-relief and so on.

The Medication checklist may be adapted and/or (partially) completed based on patient-specific data.

For example, a checklist item for a specific medication may indicate a recommended dosage for the medication based on the patient specific data. In particular examples, the generic guidance provides a formula for calculating a dosage for the medication (e.g. based on patient characteristics, such as height, age, gender and/or weight). The patient-specific data may be used to automatically execute the formula in order to calculate a recommended dosage for the medication.

As another example, a checklist item may be automatically identified as complete if the patient has already received that medication or a suitable alternative (e.g. if the patient is already sedated).

For the "Risk Assessment" section, the generic guidance may be processed, based on the patient-specific data, to automatically provide patient-specific risk information. In particular, the patient-specific risk information may identify elements of the patient data that contribute to a risk score, such as a Mallampati score (although other risk scores are envisaged, e.g. Simplified Airway Risk Index, a patient at risk (PAR) score and so on).

The generic guidance may, for example, provide a value of a risk score for each of a plurality of different possible patient conditions/parameters or identify risky patient conditions. For example, the generic guidance may indicate values of a vital sign that are considered unsafe for intubation (e.g. SpO₂ <80%). The patient-specific data may be processed to identify patient conditions contributing to the risk score (by consulting the generic guidance), and provide these patient conditions in the patient-specific guidance.

As another example, the generic guidance may indicate risk factors for a particular treatment or procedure. The patient-specific data may be used to mark or otherwise indicate which risk factors are presented by the patient.

A risk score may be automatically calculated based on the identified patient-specific data. The formula for calculating the risk score may be indicated in the generic guidance.

As illustrated, the user interface 220 may also be adapted to provide a visual representation of patient-specific data, such as real-time patient monitoring information (e.g. heart rate, respiratory rate, temperature and so on).

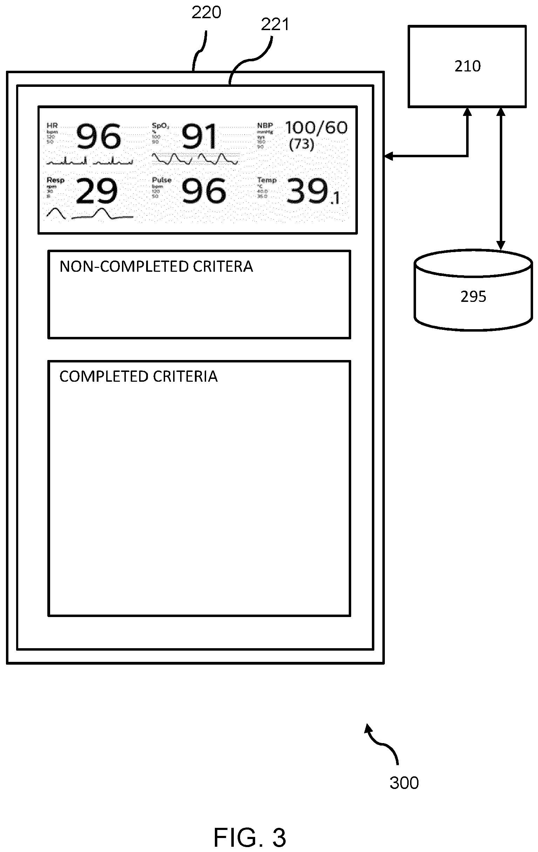

Another working example of an embodiment of the invention will be hereafter described with reference to Figure 3, which illustrates a patient-specific guidance display system 300 for displaying patient-specific guidance.

The patient-specific guidance display system 300 is identical or similar to the patient-specific guidance display system 200 of Figure 2. The difference lies in the format of the generic guidance.

In the illustrated example of Figure 3, the generic guidance is in the form of a checklist for criteria required for mobilization, i.e. targets that the patient must meet before they can be mobilized.

The generic guidance may be updated and/or processed based on patient-specific information to automatically complete, populate or mark-off criteria contained in the checklist, thus avoiding the need for a clinician to manually perform this step.

As illustrated, the visual representation of the generic guidance (at the screen 210) may be adapted so that non-complete elements of the checklist are spatially presented separately (e.g. above) completed elements of the checklist. This increases an ease of the user in identifying non-complete elements, to thereby increase an ease of using the user interface.

The clinician may then be able to complete the checklist manually (e.g. to perform steps that are not performed automatically, such as measuring a face color or consciousness level of the patient).

This information, input by the clinician, may be stored in an electronic medical record of the subject. This may, in turn, be used to adapt this (or other) generic guidance for the patient.

In some examples, the patient-specific guidance display system 200 is adapted to monitor the progress of a patient with respect to the generic guidance, i.e. using the patient-specific data, to identify when the generic guidance has been mostly completed (e.g. >70% of criteria met). This may be performed in the background, e.g. when the generic or patient-specific guidance is not visually represented on the user interface. The display system 200 may then generate an alert or notification (e.g. visually) to draw the clinician's attention to the near-completion of the generic guidance. Interacting with the alert may cause the patient-specific guidance to be visually represented at the user interface.

In some examples, when all criteria are met, the user interface may display a summary of potential follow up activities or information, such as level of mobilization, type of mobilization activities suited for this patient state, scheduling with physiotherapist and so on. This information may be generated by the processing system 210, e.g. from information stored in a database 295.

The illustrated visual representations of the patient-specific guidance are only examples, for the purposes of improved understanding, and the skilled person would be readily capable of modifying the visual representations for different forms of guidance.

The skilled person would be readily capable of adapting any above-described process or concept into a (computer-based) method of generating patient-specific guidance.

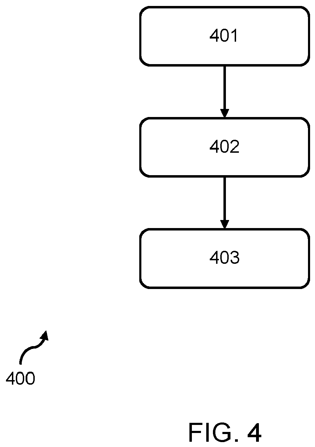

Nonetheless, for the sake of clarity, Figure 4 illustrates a flowchart of a method 400 according to an embodiment of the invention. The method 400 is for generating patient-specific guidance for treating a patient.

The method 400 comprises a step 401 of obtaining patient-specific data associated with a patient, a step 402 of obtaining generic guidance providing generic guidelines, protocols, treatment strategies and/or operating procedures for treating the patient; and a step 403 of processing the generic guidance, using one or more computer-based rules or algorithmic processes, based on the patient-specific data to generate patient-specific guidance to thereby provide patient-specific guidelines, protocols, treatment strategies and/or operating procedures for treating the patient.

The skilled person would be readily capable of developing a processing system for carrying out any herein described method or concept. Thus, each step of the flow chart may represent a different action performed by a processing system, and may be performed by a respective module of the processing system.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of computer program comprising code means for implementing any described method when said program is run on a processing system, such as a computer. Thus, different portions, lines or blocks of code of a computer program according to an embodiment may be executed by a processing system or computer to perform any herein described method. In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A method (400) of generating patient-specific guidance for treating a patient, the method comprising:
obtaining (401) patient-specific data (150) associated with a patient;
obtaining (402) generic guidance (140) providing generic guidelines, protocols, treatment strategies and/or operating procedures for treating the patient; and
processing (403) the generic guidance, using one or more computer-based rules or algorithmic processes, based on the patient-specific data to generate patient-specific guidance (105) to thereby provide patient-specific guidelines, protocols, treatment strategies and/or operating procedures for treating the patient.

2. The method of claim 1, further comprising a step of obtaining clinician information associated with a clinician attending the patient, wherein the step of processing the generic guidance comprises:
processing the generic guidance, using one or more computer-based rules or algorithmic process, based on the patient-specific data and the clinician information to generate patient and clinician specific guidance to thereby provide patient and clinician specific guidelines, protocols, treatment strategies and/or operating procedures for treating the patient.

3. The method of any of claims 1 or 2, wherein the patient-specific data (150) comprises real-time patient monitoring data.

4. The method of any of claims 1 to 3, wherein the patient-specific data (150) comprises an electronic medical record of the patient.

5. The method of any of claims 1 to 4, further comprising a step of displaying a visual representation of the patient-specific guidance.

6. The method of claim 5, further comprising, after displaying the visual representation, responding to a user input to update the patient-specific guidelines and/or the patient-specific data.

7. The method of any of claims 1 to 6, wherein the step of obtaining generic guidance comprises processing patient-specific data, using one or more computer-based rules or algorithmic processes, to identify generic guidelines for the patient.

8. The method of any of claims 1 to 7, wherein:
the step (201) of obtaining the patient-specific data comprises obtaining the patient-specific data from patient-specific data module (151) comprising a first, different database (152), a user interface (153) and/or a patient monitoring device; and
the step (202) of obtaining the generic guidance comprises obtaining the generic guidance from a second database.

9. The method of claim 8, wherein the step of obtaining the generic guidance comprises responding to a user input indicating desired generic guidance to obtain the generic guidance from the second database.

10. The method of any of claims 1 to 9, wherein the step of processing the generic guidance comprises selecting a sub-set of the generic guidelines, protocols, treatment strategies and/or operating procedures provided by the generic guidance based on the patient-specific data, to thereby generate patient-specific guidelines, protocols, treatment strategies and/or operating procedures.

11. The method of any of claims 1 to 10, wherein:
the generic guidance comprises a procedural checklist for a treatment of the patient, wherein the step of processing the generic guidance comprises populating the checklist based on the patient-specific data;
the generic guidance comprises a decision tree for aiding a clinician in making a diagnosis, wherein the step of processing the generic guidance comprises incorporating patient-specific data into the decision tree; and/or
the generic guidance comprises a proposed treatment strategy for the patient, wherein the step of processing the generic guidance comprises adapting the treatment strategy based on the patient-specific data.

12. A computer program product comprising computer program code which, when executed by one or more processing systems, cause the one or more processing systems to perform all of the steps of the method according to any of claims 1 to 11.

13. A processing system (100) for providing patient-specific guidance for treating a patient, the processing system being adapted to:
obtain (201) patient-specific data (150) associated with a patient;
obtain (202) generic guidance (140) providing generic guidelines, protocols, treatment strategies and/or operating procedures for treating the patient; and
process (203) the generic guidance, using one or more computer-based rules or algorithmic processes, based on the patient-specific data to generate patient-specific guidance (105) to thereby provide patient-specific guidelines, protocols or operating procedures for treating the patient.

14. A patient-specific guidance system comprising:
the processing system of claim 13;
a patient-specific data module (151) adapted to provide patient-specific data, wherein the patient-specific data module comprises a first database (152), a user interface (153) and/or a patient monitoring device (154);
a second, different database adapted to store generic guidance providing generic guidelines, protocols, treatment strategies and/or operating procedures for treating the patient;
wherein the processing system is adapted to obtain the patient-specific data from the patient-specific data module and the generic guidance from the second, different database.

15. A patient-specific guidance display system (1) comprising:
a processing system according to claim 13 or a patient-specific guidance system according to claim 14; and
a user interface (110) adapted to provide a visual representation of the patient-specific guidance information (105) generated by the processing system.
